# EUROPEAN PATENT SPECIFICATION

(11) **EP 2 784 153 B1**
(45) Date of publication and mention of the grant of the patent: **03.01.2018**
(21) Application number: 12852281.0
(22) Date of filing: 22.11.2012
(51) Int. Cl.: C12N 5/074, C12N 5/0735

(54) **METHOD FOR CULTURING PLURIPOTENT STEM CELL**
VERFAHREN ZUR ZÜCHTUNG PLURIPOTENTER STAMMZELLEN
PROCÉDÉ POUR LA CULTURE DE CELLULES SOUCHES PLURIPOTENTES

(30) Priority: 25.11.2011 US 201161563643 P
(43) Date of publication of application: 01.10.2014
(73) Proprietor: Kyoto University, Kyoto-shi, Kyoto 606-8501 (JP)
(72) Inventor: NAKATSUJI, Norio, Kyoto-shi Kyoto 606-8501 (JP)
(74) Representative: Vossius & Partner Patentanwälte Rechtsanwälte mbB
(86) International application number: PCT/JP2012/080364
(87) International publication number: WO 2013/077423

(56) References cited:
- WO-A1-2004/078961
- WO-A1-2010/053472
- JP-A- 2003 530 828
- JP-A- 2007 267 672
- JP-A- 2010 075 199
- STEINER D ET AL: "Derivation, propagation and controlled differentiation of human embryonic stem cells in suspension", NATURE BIOTECHNOLOGY, NATURE PUBLISHING GROUP, NEW YORK, NY, US, vol. 28, no. 4, 1 April 2010 (2010-04-01), pages 361-364,1, XP002589673, ISSN: 1087-0156, DOI: 10.1038/NBT.1616 [retrieved on 2010-03-28]
- R. Krawetz ET AL: "Large-Scale Expansion of Pluripotent Human Embryonic Stem Cells in Stirred-Suspension Bioreactors", Tissue Engineering, 1 January 2010 (2010-01-01), pages 573-582, XP055071789, Retrieved from the Internet: URL:http://online.liebertpub.com/doi/pdf/1 0.1089/ten.tec.2009.0228 [retrieved on 2013-07-17]
- ZWEIGERDT ROBERT ET AL: "Scalable expansion of human pluripotent stem cells in suspension culture", NATURE PROTOCOLS, NATURE PUBLISHING GROUP, GB, vol. 6, no. 5, 1 May 2011 (2011-05-01), pages 689-700, XP008146754, ISSN: 1750-2799, DOI: 10.1038/NPROT.2011.318 [retrieved on 2011-04-28]
- SINGH H ET AL: "Up-scaling single cell-inoculated suspension culture of human embryonic stem cells", STEM CELL RESEARCH, ELSEVIER, NL, vol. 4, no. 3, 1 May 2010 (2010-05-01), pages 165-179, XP027054727, ISSN: 1873-5061 [retrieved on 2010-05-01]
- TOMOMI G. OTSUJI ET AL: "A 3D Sphere Culture System Containing Functional Polymers for Large-Scale Human Pluripotent Stem Cell Production", STEM CELL REPORTS, vol. 2, no. 5, 1 May 2014 (2014-05-01), pages 734-745, XP055171477, ISSN: 2213-6711, DOI: 10.1016/j.stemcr.2014.03.012

## Description

### Technical Field

The present invention relates to a method of maintaining and amplifying pluripotent stem cells such as embryonic stem cells, induced pluripotent stem cells and the like. More particularly, the present invention relates to a method of maintaining and amplifying pluripotent stem cells, comprising amplifying the pluripotent stem cells to a size generally free of induction of differentiation and cell death of aggregates of the cells by suspension culture, and fragmenting the cell aggregates to a smaller size generally free of induction of the cell death and passaging same.

### Background Art

Pluripotent stem cells that can grow indefinitely without canceration and the like and have multipotency are expected to be applicable to cell transplantation treatments, drug discovery screening and the like.

Heretofore, human pluripotent stem cell line has been grown and maintained by plane culture including adhesion to feeder cells, various polymers and the like. However, a technique for culturing and growing high quality human pluripotent stem cells stably in large amounts has not been established. Particularly, the conventional method including adhesion to a culture vessel and growth by passage has a limitation as a method for preparing a large amount of pluripotent stem cells necessary for practical application. For example, an adhesive substrate material for human pluripotent stem cells, which is optimal in terms of quality and cost, has not been developed, and passage requiring complicated multistep handling generally includes steps disadvantageous from the aspects of safety and cost, such as enzyme treatment and the like.

Recently, a suspension culture method that does not require adhesive substrates has been reported and has been attracting attention (patent documents 1-5, non-patent documents 1-6). This method enables mass culture in a smaller space since it permits three-dimensional culture. However, since the suspension culture method already reported requires an enzyme treatment during passage, like the adherent culture, passage handling is complicated and cell aggregates having a uniform size is difficult. In addition, stable control of an appropriate size of the cell aggregates is not possible since cell aggregates undergo adhesion and fusion with each other to trigger cell necrosis, differentiation, and the like.

### [Document List]

### [patent documents]

patent document 1: WO 2011/058558
patent document 2: WO 2009/116951
patent document 3: WO 2008/120218
patent document 4: WO 2008/015682
patent document 5: WO 2007/002086

### [non-patent documents]

non-patent document 1: M. Amit et al., Nat. Protoc., 325, 572-579 (2011)
non-patent document 2: R. Zweigerdt et al., Nat. Protoc., 318, 689-700 (2011)
non-patent document 3: D. Steiner et al., Nat. Biotechnol., 28, 361-364 (2010)
non-patent document 4: M. Amit et al., Stem Cell Rev. and Rep., 6, 248-259 (2010)
non-patent document 5: H. Singh et al., Stem Cell Res.,4, 165-179 (2010)
non-patent document 6: A.K. Chen et al., Stem Cell Res., 7, 97-111 (2011)

### Summary of the Invention

### Problems to be Solved by the Invention

An object of the present invention is to provide a novel culture method of pluripotent stem cells that solves the problems of the suspension culture method already reported. That is, a first problem of the present invention is to provide a novel passage method that does not require an enzyme treatment and is capable of fragmentation into cell aggregates having a uniform size. Moreover, a second problem of the present invention is to provide a novel suspension culture method that can lower the possibility of adhesion and fusion of cell aggregates.

### Means of Solving the Problems

Generally, ES cell aggregates having a nearly spherical form in suspension culture tend to show apoptosis when the size of the cell aggregates is too small. Conversely, when the size of the cell aggregates is too large, problems occur such as the initiation of differentiation and necrosis of central cells. Therefore, the present inventor first studied the size of cell aggregates suitable for the maintenance and amplification of human embryonic stem cells (ES cell). As a result, the present inventor has clarified that ES cell aggregates show good growth while maintaining pluripotency when the diameter is within the range of about 80 - about 250 µm. Therefore, it is considered that ES cell can be maintained and amplified most efficiently when ES cell aggregates can be grown by the suspension culture method to have a diameter of about 250 um, fragmented into uniform cell aggregates having a diameter of about 80 µm and passaged.

Therefore, the present inventor conducted intensive studies of a means to conveniently fragment ES cell aggregates into a uniform size without using an enzyme treatment, and found that large cell aggregates can be fragmented by simply passing the cell suspension through a mesh-like filter, and uniform cell aggregates having a smaller size can be prepared. Although an operation including passing the cells dispersed by an enzyme treatment through a mesh to obtain cells with a uniform size by removing large cell aggregates that remained without dissociation during the enzyme treatment has heretofore been performed, cell aggregates larger than the pore size of the mesh are considered to remain without passing the mesh. Thus, a novel passage method including forming cell aggregates with a smaller size by easily fragmenting cell aggregates by passing the cell aggregates through a mesh with a smaller pore size than the size of the aggregates is provided.

Then, the present inventor studied a means to lower the possibility of adhesion and fusion of cell aggregates during culture. As a method for preventing adhesion and fusion of cell aggregates, for example, a method involving standing the aggregates still without moving after dispersion has been conventionally employed. However, such method makes culture handling highly difficult since microscope observation is not possible and the like. Alternatively, a method involving constantly shaking the cell liquid has also been used. However, adverse influences occur such as complicated culture method, cell damage due to a shearing force of the cell liquid and the like, and an effective means for preventing adhesion and fusion of floating cell aggregates has not been found. Thus, the present inventor imparted suitable viscosity to the culture medium by adding a polymer compound without cytotoxicity at a given concentration, thus succeeding in the prevention of adhesion and fusion of floating cell aggregates by suppressing movement of floating cell aggregate spheres and close adhesion of the spheres.

The present inventor has solved the problems in the suspension culture method of pluripotent stem cells by combining the above-mentioned two novel methods and strikingly improved the amplification efficiency of ES cells, which resulted in the completion of the present invention.

Accordingly, the present invention provides the following.
[1] A method of maintaining and amplifying pluripotent stem cells, comprising repeating the following steps:
   (i) suspension culturing pluripotent stem cells until cell aggregates have an average diameter of about 200 - about 300 µm,
   (ii) fragmenting the cell aggregates obtained by step (i) into cell aggregates having a uniform average diameter of about 80 - about 120 µm.
[2] The method of the above-mentioned [1], wherein the suspension culture of the pluripotent stem cells in step (i) is performed until the cell aggregates have an average diameter of about 250 µm, and the fragmentation in step (ii) affords uniform cell aggregates having an average diameter of about 80 µm.
[3] The method of the above-mentioned [1] or [2], wherein the fragmentation in step (ii) is performed by passing the cell aggregates through a mesh.
[4] The method of the above-mentioned [3], wherein the pore size of the mesh is about 30 - about 70 µm, preferably about 40 - about 60 µm, more preferably about 50 µm.
[5] The method of any of the above-mentioned [1] - [4], wherein the culture in step (i) is performed in a medium containing a water-soluble polymer component having a viscosity that does not cause adhesion of cell aggregates.
[6] The method of the above-mentioned [5], wherein the water-soluble polymer is selected from polysaccharide or ether thereof, a synthetic hydrogel polymer and a biopolymer, and artificial polymers mimicking them.
[7] The method of the above-mentioned [5], wherein the water-soluble polymer is methylcellulose or a temperature rise-type thermosensitive hydrogel.
[8] The method of any of the above-mentioned [1] - [7], wherein the pluripotent stem cell is an ES cell or an iPS cell.
[9] The method of any of the above-mentioned [1] - [8], wherein the pluripotent stem cell is derived from human.

### Effect of the Invention

According to the passage method of the present invention, following growth of cell aggregates having a uniform small size by cell proliferation, a single convenient step of passing a suspension of the cell aggregates through a mesh at a stage which the cell aggregates have an appropriate size before the initiation of cell necrosis and differentiation enables re-fragmentation into cell aggregates having a uniform small size and passage thereof, which is extremely advantageous from the aspects of safety and cost since the step does not require an enzyme treatment unlike the conventional methods. Moreover, the size of the cell aggregates after fragmentation is rich in uniformity, and facilitates control of the size of the cell aggregates to fall within an optimal range, which is the problem of the conventional suspension culture.

Moreover, according to the culture method after passage of the present invention, adhesion and fusion of cell aggregates can be easily prevented by suppressing movement and close adhesion of floating cell aggregates. Therefore, necrosis and initiation of differentiation of the cell aggregates due to a size increase, which is the problem of the conventional suspension culture, can be suppressed and ES cell can be efficiently maintained and amplified. In addition, a complicated operation of standing, continued shaking accompanied by the risk of cell damage, and the like, becomes unnecessary, and the growth of cell aggregates and culture state can be monitored by microscopic observation throughout the whole period of passage and culture.

### Brief Description of the Drawings

Fig. 1 shows the results of suspension culture (at passage 4) of human ES cells (KhES-1 cell line).
Fig. 2 shows the FACS analysis results showing the expression of pluripotent stem cell marker in human ES cell after 11 passages.
Fig. 3 shows the results of immunostaining showing the expression of a stem cell marker in a frozen section of human ES cells (KhES-1 cell line) after 11 passages.
Fig. 4 shows human ES cell colony produced on a feeder cell by adherent culture of human ES cells (KhES-1 cell line) after 18 passages.
Fig. 5 shows the results of immunostaining showing the expression of various pluripotent stem cell markers in human ES cell colony produced on a feeder cell by adherent culture of human ES cells (KhES-1 cell line) after 18 passages.
Fig. 6 shows the analysis results of the karyotype of human ES cells (KhES-1 cell line) after 17 passages.
Fig. 7 shows the results of suspension culture of human iPS cells (IMR90-1 cell line).
Fig. 8 shows the results of immunostaining showing the expression of a stem cell marker in a frozen section of human iPS cells (253G1 cell line) after 22 passages.
Fig. 9 shows the results of consideration of the differentiation ability of 253G1 and KhES-1 cells into cardiomyocytes in vitro after long-term passage.
Fig. 10 shows the results of consideration of the differentiation ability of 253G1 cells into neurons in vitro after long-term passage.
Fig. 11 shows daily changes in the sphere form of 253G1 cells after passage.
Fig. 12 shows the results of consideration of the effect of mesh size on the cell proliferation in the passage using 253G1 cells. The vertical axis shows a fold increase relative to the cell number immediately after passage.
Fig. 13 shows the results of consideration of the effect of mesh size on the sphere form in the passage using 253G1 cells. Scale bar: 100 µm
Fig. 14 shows the effect of methylcellulose in suspension sphere culture of 253G1 cells.

### Description of Embodiments

The Description of Embodiments is explained in detail in the following.

The present invention provides a novel and useful method for the maintenance and amplification of pluripotent stem cells. The method characteristically includes repeating the following steps:
(i) suspension culturing pluripotent stem cells until cell aggregates have an average diameter of about 200 - about 300 µm,
(ii) fragmenting the cell aggregates obtained by step (i) into cell aggregates having a uniform average diameter of about 80 - about 120 µm.

The pluripotent stem cell to which the method of the present invention can be applied is not particularly limited as long as it is an undifferentiated cell possessing a "self-renewal ability" that enables it to proliferate while retaining the undifferentiated state, and "pluripotency" that enables it to differentiate into all the three primary germ layers of the embryo. Examples thereof include ES cell, induced pluripotent stem cell (iPS cell), embryonic germ (EG) cell derived from a primordial germ cell, multipotent germline stem (mGS) cell isolated in the process of establishment and culture of GS cell from testis tissue, multipotent adult progenitor cell (MAPC) isolated from bone marrow and the like. The ES cell may be produced from a somatic cell by nuclear reprogramming. Preferred are ES cells or iPS cells. The method of the present invention is applicable to any mammalian species for which any pluripotent stem cell line has been established or can be established. Examples thereof include humans, mice, monkeys, pigs, rats, dogs and the like. Preferred are human and mice, more preferred is human.

### I. Preparation of pluripotent stem cell

ES cells can be maintained by passage culture using a culture medium added with substances such as leukemia inhibitory factor (LIF), basic fibroblast growth factor (bFGF) and the like. Methods of establishment and maintenance of human and monkey ES cells are described in, for example, USP5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279;H. Kawasaki et al. (2002), Proc. Natl. Acad. Sci. USA, 99:1580-1585;Klimanskaya I, et al. (2006), Nature. 444:481-485 and the like.

Using, as a culture medium for preparing ES cells, for example, a DMEM/F-12 culture medium (or, synthetic medium: mTeSR, Stem Pro and the like) supplemented with 0.1 mM 2-mercaptoethanol, 0.1 mM nonessential amino acids, 2 mM L-glutamic acid, 20% KSR and 4 ng/ml bFGF, human ES cells can be maintained under wet atmosphere at 37°C, 2% CO₂/98% air (O. Fumitaka et al. (2008), Nat. Biotechnol., 26:215-224). In addition, ES cells require passage every 3 - 4 days, and the passage in this case can be performed using, for example, 0.25% trypsin and 0.1 mg/ml collagenase IV in PBS containing 1 mM CaCl₂ and 20% KSR.

ES cells can be generally selected by the Real-Time PCR method using the expression of a gene marker such as alkaline phosphatase, Oct-3/4, Nanog and the like as an index. Particularly, for selection of human ES cell, expression of a gene marker such as OCT-3/4, NANOG, ECAD and the like can be used as an index (E. Kroon et al. (2008), Nat. Biotechnol., 26:443-452).

As for human reference ES cell line, for example, WA01 (H1) and WA09(H9) are available from WiCell Research Institute, and KhES-1, KhES-2 and KhES-3 are available from Institute for Frontier Medical Sciences, Kyoto University (Kyoto, Japan).

Spermatogonial stem cell is a pluripotent stem cell derived from the testis, which becomes the origin for spermatogenesis. This cell can be differentiation induced into various lines of cells, like ES cells and shows properties of, for example, generation of a chimeric mouse by transplantation into a mouse blastocyst and the like (M. Kanatsu-Shinohara et al. (2003) Biol. Reprod., 69:612-616; K. Shinohara et al. (2004), Cell, 119:1001-1012). It is self-renewable in a culture medium containing a glial cell line-derived neurotrophic factor (GDNF), can produce a spermatogonial stem cell by repeating passages under culture conditions similar to those for ES cells (Masanori Takehashi et al., (2008), Experimental Medicine, Vol. 26, No. 5(Suppl.), pp. 41 - 46, YODOSHA (Tokyo, Japan)).

Embryonic germ cell is a cell having pluripotency similar to that of ES cells, which is established from a primordial germ cell at the prenatal period. It can be established by culturing a primordial germ cell in the presence of a substance such as LIF, bFGF, a stem cell factor and the like (Y. Matsui et al. (1992), Cell, 70:841-847; J.L. Resnick et al. (1992), Nature, 359:550-551).

Induced pluripotent stem (iPS) cell is an artificial stem cell derived from a somatic cell, which can be produced by introducing a specific reprogramming factor in the form of a DNA or protein into a somatic cell, and show almost equivalent property (e.g., pluripotent differentiation and proliferation potency based on self-renewal) as ES cells (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO 2007/069666). The reprogramming factor may be constituted with a gene specifically expressed by ES cell, a gene product or non-coding RNA thereof, a gene playing an important role for the maintenance of undifferentiation of ES cell, a gene product or non-coding RNA thereof, or a low molecular weight compound. Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fb15, ERas, ECAT15-2, Tel1, beta-catenin, Lin28b, Sall1, Sall4, Esrrb, Nr5a2, Tbx3, Glis1 and the like. These reprogramming factors may be used alone or in combination. Examples of the combination of the reprogramming factors include combinations described in WO 2007/069666, WO 2008/118820, WO 2009/007852, WO 2009/032194, WO 2009/058413, WO 2009/057831, WO 2009/075119, WO 2009/079007, WO 2009/091659, WO 2009/101084, WO 2009/101407, WO 2009/102983, WO 2009/114949, WO 2009/117439, WO 2009/126250, WO 2009/126251, WO 2009/126655, WO 2009/157593, WO 2010/009015, WO 2010/033906, WO 2010/033920, WO 2010/042800, WO 2010/050626, WO 2010/056831, WO 2010/068955, WO 2010/098419, WO 2010/102267, WO 2010/111409, WO 2010/111422, WO 2010/115050, WO 2010/124290, WO 2010/147395, WO 2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, Maekawa M, et al. (2011), Nature. 474:225-9.

The above-mentioned reprogramming factor also includes factors used to enhance establishment efficiency, such as histone deacetylase (HDAC) inhibitors [e.g., low-molecular inhibitors such as valproic acid (VPA), trichostatin A, sodium butyrate, MC 1293, and M344, nucleic acid-based expression inhibitors such as siRNAs and shRNAs against HDAC (e.g., HDAC1 siRNA Smartpool® (Millipore), HuSH 29mer shRNA Constructs against HDAC1 (OriGene) and the like), and the like], MEK inhibitor (e.g., PD184352, PD98059, U0126, SL327 and PD0325901), Glycogen synthase kinase-3 inhibitor (e.g., Bio and CHIR99021), DNA methyl transferase inhibitors (e.g., 5-azacytidine), histone methyl transferase inhibitors [e.g., low-molecular inhibitors such as BIX-01294, and nucleic acid-based expression inhibitors such as siRNAs and shRNAs against Suv39h1, Suv39h2, SetDBl and G9a], L-channel calcium agonist (for example, Bayk8644), butyric acid, TGFβ inhibitor or ALK5 inhibitor (e.g., LY364947, SB431542, 616453 and A-83-01), p53 inhibitor (for example, siRNA and shRNA against p53), ARID3A inhibitor (e.g., siRNA and shRNA against ARID3A), miRNA such as miR-291-3p, miR-294, miR-295, mir-302 and the like, Wnt Signaling (for example, soluble Wnt3a), neuropeptide Y, prostaglandins (e.g., prostaglandin E2 and prostaglandin J2), hTERT, SV40LT, UTF1, IRX6, GLIS1, PITX2, DMRTB1 and the like. In the present specification, these factors used for enhancing the establishment efficiency are not particularly distinguished from the reprogramming factor.

When the reprogramming factor is in the form of a protein, it may be introduced into a somatic cell by a method, for example, lipofection, fusion with cell penetrating peptide (e.g., TAT derived from HIV and polyarginine), microinjection and the like.

On the other hand, when it is in the form of a DNA, it may be introduced into a somatic cell by the method using, for example, vector of virus, plasmid, artificial chromosome and the like, lipofection, liposome, microinjection and the like. Examples of the virus vector include retrovirus vector, lentivirus vector (Cell, 126, pp.663-676, 2006; Cell, 131, pp.861-872, 2007; Science, 318, pp.1917-1920, 2007), adenovirus vector (Science, 322, 945-949, 2008), adeno-associated virus vector, Sendai virus vector (WO 2010/008054) and the like. Examples of the artificial chromosome vector include human artificial chromosome (HAC), yeast artificial chromosome (YAC), bacterial artificial chromosome (BAC, PAC) and the like. As the plasmid, plasmids for mammalian cells can be used (Science, 322:949-953, 2008). The vector can contain regulatory sequences of promoter, enhancer, ribosome binding sequence, terminator, polyadenylation site and the like so that a nuclear reprogramming substance can be expressed and further, where necessary, a selection marker sequence of a drug resistance gene (for example, kanamycin resistance gene, ampicillin resistance gene, puromycin resistance gene and the like), thymidine kinase gene, diphtheria toxin gene and the like, a reporter gene sequence of green fluorescent protein (GFP), β glucuronidase (GUS), FLAG and the like, and the like. Moreover, the above-mentioned vector may have a LoxP sequence before and after thereof to simultaneously cut out a gene encoding a reprogramming factor or a gene encoding a reprogramming factor bound to the promoter, after introduction into a somatic cell.

When it is in the form of an RNA, for example, it may be introduced into a somatic cell by a method of lipofection, microinjection and the like, and RNA incorporating 5-methylcytidine and pseudouridine (TriLink Biotechnologies) may be used to suppress degradation (Warren L, (2010) Cell Stem Cell. 7:618-630).

Examples of the culture medium for inducing iPS cell include 10 - 15% FBS-containing DMEM, DMEM/F12 or DME culture medium (these culture media can further contain LIF, penicillin/streptomycin, puromycin, L-glutamine, nonessential amino acids, β-mercaptoethanol and the like as appropriate) or a commercially available culture medium [for example, culture medium for mouse ES cell culture (TX-WES culture medium, Thromb-X), culture medium for primate ES cell culture (culture medium for primate ES/iPS cell, Reprocell), serum-free medium (mTeSR, Stemcell Technologies)] and the like.

Examples of the culture method include contacting a somatic cell with a reprogramming factor on 10% FBS-containing DMEM or DMEM/F12 culture medium at 37°C in the presence of 5% CO₂ and culturing for about 4 - 7 days, thereafter reseeding the cells on feeder cells (e.g., mitomycin C-treated STO cells, SNL cells etc.), and culturing the cells in a bFGF-containing culture medium for primate ES cell culture from about 10 days after the contact of the somatic cell and the reprogramming factor, whereby iPS-like colonies can be obtained after about 30 - about 45 days or longer from the contact.

Alternatively, the cells are cultured on feeder cells (e.g., mitomycin C-treated STO cells, SNL cells etc.) at 37°C in the presence of 5% CO₂ in a 10% FBS-containing DMEM culture medium (which can further contain LIF, penicillin/streptomycin, puromycin, L-glutamine, nonessential amino acids, β-mercaptoethanol and the like as appropriate), whereby ES-like colonies can be obtained after about 25 - about 30 days or longer. Desirably, a method using a somatic cell itself to be reprogrammed, or an extracellular substrate (e.g., Laminin (WO 2009/123349) and Matrigel (BD)), instead of the feeder cells (Takahashi K, et al. (2009), PLoS One. 4:e8067 or WO 2010/137746), can be mentioned.

Besides the above, a culture method using a serum-free medium can also be recited as an example (Sun N, et al. (2009), Proc Natl Acad Sci U S A. 106:15720-15725). Furthermore, to enhance establishment efficiency, an iPS cell may be established under hypoxic conditions (oxygen concentration of not less than 0.1% and not more than 15%) (Yoshida Y, et al. (2009), Cell Stem Cell. 5:237-241 or WO 2010/013845).

The culture medium is exchanged with a fresh culture medium once a day during the above-mentioned cultures, from day 2 from the start of the culture. While the cell number of the somatic cells used for nuclear reprogramming is not limited, it is about 5×10³ - about 5×10 cells per 100 cm² culture dish.

The iPS cell can be selected based on the shape of the formed colony. When a drug resistance gene which is expressed in association with a gene (e.g., Oct3/4, Nanog) expressed when a somatic cell is reprogrammed is introduced as a marker gene, an established iPS cell can be selected by culturing in a culture medium (selection culture medium) containing a corresponding drug. When the marker gene is a fluorescent protein gene, iPS cell can be selected by observation with a fluorescence microscope, when it is a luminescent enzyme gene, iPS cell can be selected by adding a luminescent substrate, and when it is a chromogenic enzyme gene, iPS cell can be selected by adding a chromogenic substrate.

### II. Suspension culture of pluripotent stem cells (step (i))

Pluripotent stem cells prepared as mentioned above are subjected to suspension culture until the cell aggregates have an average diameter of about 200 - about 300 µm. Here, "about" means ±10% is acceptable. When the diameter of cell aggregates exceeds 300 µm, a microenvironment is formed due to an influence of cytokine and the like secreted by the cells, which induces differentiation. In addition, since necrosis occurs in the central part of the cell aggregates, the recovery rate of the viable cells becomes low. On the other hand, the lower limit of the average diameter of the cell aggregates is not particularly limited as long as it is larger than the average diameter of the cell aggregates when the suspension culture is started (at the time of passage in suspension culture after passage). The culture is preferably continued up to not less than about 200 µm, when the yield of the pluripotent stem cells is considered.

As the medium for suspension culture, one having a similar composition as that of the medium for adherent culture exemplified in the above-mentioned I. can be used. To prevent movement of cell aggregates and close adhesion of cell aggregates, preferably, an appropriate viscosity is desirably conferred to the medium. Here, an appropriate viscosity means a viscosity of the level preventing adhesion of cell aggregates without preventing medium exchange.

While the means to confer viscosity to the medium is not particularly limited, for example, a water-soluble polymer is added at a suitable concentration to the medium. As the water-soluble polymer, any water-soluble polymer can be used as long as it can impart the above-mentioned appropriate viscosity to the medium, and does not exert an adverse influence on the cell (no cytotoxicity) in the concentration range capable of imparting the viscosity. Examples thereof include polysaccharides such as cellulose, agarose and the like, polysaccharide ethers such as methylcellulose, ethylcellulose, hydroxyethylcellulose, hydroxypropylcellulose, hydroxyethylmethylcellulose, hydroxypropylmethylcellulose, hydroxyethylethylcellulose, hydroxypropylethylcellulose, ethylhydroxyethylcellulose, dihydroxypropylcellulose, hydroxyethylhydroxypropylcellulose and the like, synthetic polymers such as polyacrylamide, polyethylene oxide, and polyvinylpyrrolidone, ethylene glycol/propylene glycol copolymer, polyethyleneimine polyvinyl methylether, polyvinyl alcohol, polyacrylic acid, maleic acid copolymer and the like, biopolymers such as collagen, gelatin, hyaluronic acid, dextran, alginic acid, carrageenan, starch and the like, and artificial polymers mimicking them (e.g., elastin-like peptide and the like). Preferably, these water-soluble polymers may be used alone or as a mixture of several kinds of water-soluble polymers. In addition, a copolymer of these water-soluble polymers may also be used. Preferably, methylcellulose, polyethylene glycol, polyvinylpyrrolidone, carboxymethylcellulose or a mixture thereof, more preferably methylcellulose, can be used.

The concentration of the water-soluble polymer to be added to the medium varies depending on the kind of the water-soluble polymer, and the kind, culture temperature and the like of the pluripotent stem cell line to be cultured. For example, when methylcellulose is added to the medium to confer viscosity, the concentration of methylcellulose is, for example, higher than 0.2 w/v% and lower than 1.0 w/v%. When the concentration of methylcellulose is not more than 0.2 w/v%, the viscosity is too low to afford a desired effect, and when the concentration of methylcellulose is not less than 1.0 w/v%, handleability during centrifugation becomes unpreferably poor. Preferably, the methylcellulose concentration in suspension culture of human ES cell line KhES-1 and human iPS cell line 253G1 is 0.26 - 0.35 w/v%, particularly preferably about 0.28 - 0.30 w/v%. In addition, the methylcellulose concentration in suspension culture of human ES cell line H9 is preferably about 0.3 - about 0.9 w/v%, more preferably about 0.45 - about 0.75 w/v%, particularly preferably about 0.6 w/v%. Even when other water-soluble polymer is used, those of ordinary skill in the art can select an appropriate concentration of the water-soluble polymer to achieve the above-mentioned appropriate medium viscosity.

In another preferable embodiment, a temperature rise-type thermosensitive hydrogel can be used as the water-soluble polymer. The "temperature rise-type thermosensitive hydrogel" means a hydrogel which is liquid at low temperature, gelates as the temperature rises, and re-solates or shows reverse sol-gel phase transition when cooled to room temperature. Examples of the temperature rise-type thermosensitive hydrogel include, but are not limited to, trade name "Mebiol (registered trade mark) gel" series (Mebiol Inc.) showing a gel transition temperature of 27-32°C, and the like. When a temperature rise-type thermosensitive hydrogel is used, the hydrogel is added at a concentration capable of imparting a viscosity sufficient to prevent movement of floating cell aggregates and close adhesion of cell aggregates, the aggregates are grown by suspension culture until they have a size suitable for passage, and cooled to a gel transition temperature or lower to solate the culture medium, which is then centrifuged to easily recover the cells.

The culture vessel to be used for suspension culture is not particularly limited as long as it is a non-adhesive culture vessel and, for example, flask, flask for tissue culture, dish, petri dish, dish for tissue culture, multidish, microplate, microwell plate, multiplate, multiwell plate, chamber slide, petri dish, tube, tray, culture bag, and roller bottle can be mentioned.

Adherent cultured pluripotent stem cells are dissociated by an enzyme treatment, plated in the above-mentioned culture vessel at a cell density of; for example, about 0.5 - about 50×10⁴ cells/cm², preferably about 1 - about 10×10⁴ cells/cm², and cultured, for example, under an atmosphere of about 1 - about 10%, preferably about 2 - about 5% CO₂ in a CO₂ incubator at about 30 - about 40°C, preferably about 37°C, for 1 - 7 days, preferably 3 - 6 days, more preferably 4 - 5 days. The medium is desirably exchanged with a fresh medium every 1 - 2 days.

For example, when the average diameter of the cell aggregates of pluripotent stem cells at the start of suspension culture is about 80 µm and the aggregates are to be grown to about 250 µm, the cell number of the cell aggregates needs to be amplified to about 3³=27 fold. For example, since human ES cells divide once in about 24 hr, 4 - 5 days' culture will result, by calculation, in the growth to the desired size. Since the cell growth may not be constant depending on various culture conditions, the passage can also be performed at an appropriate timing while monitoring the size of the cell aggregates. According to the culture method of the present invention, the culture vessel does not need to be stood still during the culture period, since the medium is conferred with viscosity. Thus, the size of the cell aggregates can be monitored by microscope observation.

Since the cell aggregates are comparatively small immediately after the passage treatment, a ROCK inhibitor is desirably added to the medium to suppress cell death. As the ROCK inhibitor, one known per se can be used as appropriate and, for example, Y-27632 and the like can be mentioned. The concentration of the ROCK inhibitor to be added can be appropriately determined within the range generally employed, and the inhibitor can be added to the medium at a concentration of, for example, about 10 µM. It is not preferable for the cells to contain a ROCK inhibitor in a medium for a long period. Thus, the medium is desirably replaced by a medium free of a ROCK inhibitor, at the first medium exchange (e.g., one day later).

In this manner, movement of floating cell aggregates and adhesion and fusion of cell aggregates can be prevented by increasing the viscosity of the medium, and pluripotent stem cells can be maintained and amplified in cell aggregates having a uniform size, while suppressing the initiation of differentiation and/or cell death.

### III. Passage of pluripotent stem cells (step (ii))

The cell aggregates of pluripotent stem cells having a uniform size (average diameter of about 200 - about 300 µm), which are obtained in step (i), are then fragmented into uniform small cell aggregates having an average diameter of about 80 - about 120 µm, and passaged. Here, "about" means ±20% is acceptable. When the cell aggregates are fragmented to have an average diameter of not more than 50 µm, the cells unpreferably develop cell death such as apoptosis and the like. While the upper limit of the average diameter after fragmentation is not particularly limited, since the efficiency of amplification by the next suspension culture after passage becomes lower as the size grows bigger, it is preferably not more than about 120 µm, particularly preferably about 80 µm.

While a method for fragmenting the cell aggregates into uniform cell aggregates with a smaller size is not particularly limited as long as it does not contain cell dissociation by an enzyme treatment, it is preferably a method including passing a cell suspension through a mesh. The mesh to be used here is not particularly limited as long as it is sterilizable and, for example, nylon mesh, metal mesh such as stainless and the like, and the like can be mentioned. The pore size of the mesh only needs to be a size that achieves the average diameter of the cell aggregates after fragmentation of about 80 - about 120 µm, preferably about 80 µm. For example, in the case of a nylon mesh, the pore has a size of about 20 - about 100 µm, preferably about 30 - about 70 µm, more preferably about 40 - about 60 µm, particularly preferably about 50 µm. While the shape and the like of the mesh line are not particularly limited, the mesh line is required to have a width and a shape least damaging to the cell. For example, since a metal mesh such as stainless and the like can easily narrow the width of the mesh line (e.g., 35-30 µm etc.), it is expected to afford better growth in the suspension culture after passage.

As a method for passing a cell suspension through a mesh, a method including recovering a cell suspension from a culture vessel and passing same through a mesh by using Pipetman can be mentioned. By simply passing a cell suspension, the cell aggregates are mechanically fragmented while automatically passing through the mesh to form uniform small cell aggregates. More specifically, for example, a cell suspension is recovered in a tube from a culture vessel, the medium is removed, a medium containing a ROCK inhibitor is added, cells in an amount necessary for passage are transferred to another tube containing the same medium, and the cell suspension thereof is passed through a sterilized mesh placed on the tube with Pipetman. The cell aggregates fragmented by being passed through the mesh are recovered in the tube. In this way, cell aggregates of pluripotent stem cells having a uniform size (average diameter of about 80 - about 120 µm) can be obtained. The obtained cell suspension is plated on a suitable non-adhesive culture vessel, and the above-mentioned step (i) is performed again, whereby pluripotent stem cells can be maintained and amplified.

By repeating steps (i) and (ii) in this manner, a large amount of pluripotent stem cells can be stably amplified without inducing differentiation and cell death, and a sufficient amount of pluripotent stem cells can be supplied as a source of differentiated cells for a cell transplantation treatment and drug screening.

The present invention is explained in more detail in the following by referring to Examples, which are mere exemplifications and do not limit the present invention in any way.

### Examples

### [Materials]

### Basal medium and stock solution of medium additives

- mTeSR1 (medium of known composition; purchased from Stem Cell Technology, model number 05850)
- 3% methylcellulose stock solution (in IMDM medium) (purchased from R&D, model number HSC001)

Methylcellulose is melted before use, re-frozen in 2.0 ml volume, and immediately preserved at -20°C.
- 10 mM Y-27632 stock solution (in Ca, Mg-free PBS) (Rock inhibitor; purchased from Sigma, model number Y0503)

### Culture dish

- Ultra Low Cluster Plate, 6-well with cover (purchased from Corning, model number Costar 3471)
- 35 mm petri dish (purchased from BD, model number 351008)

### Mesh for passage

- CellTrics filters having pore sizes of 10, 20, 30 and 50 µm (purchased from PARTEC, model numbers 06-04-004-2324, 06-04-004-2325, 06-04-004-2326 and 06-04-004-2327, respectively)
- cell strainer having a pore size of 40 µm (purchased from BD, model number 352340)

### Medium 1 (for passage culture; containing Rock inhibitor)

mTeSR1 10 ml

3% sterilized methylcellulose 1 ml (final concentration 0.28%)*

10 mM Y-27632 11 µl (final concentration 10 µM)

*Depending on the experiment, media having final concentrations of 0 (no addition), 0.25, 0.3 and 0.5% were also used.

### Medium 2 (for medium exchange; Rock inhibitor-free)

mTeSR1 10 ml

3% sterilized methylcellulose 1 ml (final concentration 0.28%)*

*Depending on the experiment, media having final concentrations of 0 (no addition), 0.25, 0.3 and 0.5% were also used.

### Human pluripotent stem (hPS) cell line

Reference human embryonic stem cell line (hES cell line): KhES-1 (available from Institute for Frontier Medical Sciences, Kyoto University)
- human induced pluripotent stem cell line (hiPS cell line):
   IMR90-1 (available from WiCell, Wisconsin, USA)
      253G1 (available from RIKEN BIORESOURCE CENTER CELL BANK)

### [Method]

### Suspension sphere culture and passage culture

A well or dish was moved to draw a circle to collect hPS cell spheres in the center of the well or dish, and the cell spheres were transferred to 15 ml tube A. mTeSR (2.5 ml) was added to the well or dish, and the remaining aggregates were collected in 15 ml tube A. Tube A was centrifuged, and the supernatant was removed. The spheres were resuspended in 0.5 ml medium 1. Another new 15 ml tube B was prepared, and 3 ml of medium 1 was added. The sphere suspension was gently pipetted three times with a 200 µl pipette, and a necessary amount of sphere suspension was taken in tube B. The split ratio at passage was 1:3 - 1:4 for the early passage (passage number 1 - 2), thereafter 1:8 - 1:12.

Then, the sphere suspension was passed through a sterilized CellTrics filter or cell strainer (mounted on the tip of a 5 ml tube). The sphere suspension was gently pipetted three times with a 5 ml pipette, and plated on the well or dish. The well or dish was cultured in a CO₂ incubator under conventional conditions of 37°C, 5% CO₂. On day 1 and day 3, the medium was exchanged with medium 2 heated in advance.

Reference example 1: Preliminary consideration of methylcellulose

### concentration

As a preliminary experiment, hES cell line KhES-1 was cultured in medium 1 and medium 2 containing 0.25%, 0.28% or 0.5% (w/v) methylcellulose. Fusion of cell spheres could be prevented most efficiently in the concentration of 0.28 w/v% methylcellulose (fusion rates of 1.0%, 1.1%, 1.3% and 1.6% in 4 measurements; 8-13 are fusion spheres in 722-978 spheres in total). In the experiments thereafter, 0.28 w/v% methylcellulose-containing medium was used unless otherwise specified.

### Example 2: Preliminary consideration of mesh size

As a mesh to be used at passage, a CellTrics filter having a pore size of 50 µm and a cell strainer having a pore size of 40 µm were compared. Since the mesh having a pore size of 50 µm showed superior cell proliferation effect, in later experiments, a mesh having a pore size of 50 µm was used unless otherwise specified.

### Reference

### Example 3: Suspension sphere culture of human ES cell line KhES-1 (up to 20 passages)

Using medium 1 and medium 2 containing 0.28 w/v% methylcellulose, and CellTrics filter having a pore size of 5.0 µm as a mesh, suspension sphere culture of KhES-1 cell line was performed up to 20 passages according to the procedure described in the aforementioned [Method]. The results are shown in Table 1.

**[Table 1-1]**

| **Record of suspension sphere culture of KhES-1 cells in long-term passage** | | | | | |
|---|---|---|---|---|---|
| split ratio | passage number (left) and sphere number (right) | number of days after passage | average sphere diameter (µm) | standard deviation of sphere diameter (µm) | number of spheres measured |
| | P1 | 1d | 88.21 | ±13.84 | n=37 |
| | | 3d | 146.4 | ±22.38 | n=90 |
| | | 5d | 232.36 | ±25.73 | n=101 |
| X 2 | P2 | 1d | 106.46 | ±17.41 | n=27 |
| | | 3d | 166.63 | ±29.22 | n=75 |
| | | 5d | 235.6 | ±27.19 | n=88 |
| X 3 | P3 | 1d | 95.93 | ±19.20 | n=112 |
| | | 3d | 167.26 | ±32.79 | n=155 |
| | 908 | 5d | 247.41 | ±33.17 | n=98 |
| X 6 | P4 | 1d | 93.7 | ±15.55 | n=77 |
| | | 3d | 174.17 | ±34.23 | n=127 |
| | 748 | 5d | 238.7 | ±38.00 | n=120 |
| X 8 | P5 | 1d | 109.46 | ±19.62 | n=142 |
| | | 3d | 177.76 | ±29.13 | n=128 |
| | 820 | 5d | 236.35 | ±40.00 | n=107 |
| X 9 | P6 | 1d | 94.32 | ±13.77 | n=104 |
| | | 3d | 171.23 | ±23.08 | n=125 |
| | 809 | 5d | 227.02 | ±35.30 | n=167 |
| X 10 | P7 | 1d | 98.08 | ±17.72 | n=264 |
| | | 3d | 162.68 | ±24.98 | n=166 |
| | 713 | 5d | 226.46 | ±32.07 | n=139 |
| X 10 | P8 | 1d | 104.25 | ±21.92 | n=181 |
| | | 3d | 180.14 | ±31.00 | n=142 |
| | 231 | 5d | 236.51 | ±40.08 | n=110 |
| X 3 | P9 | 1d | 105.79 | ±19.20 | n=217 |
| | | 3d | 188.53 | ±29.01 | n=145 |
| | 451 | 5d | 241.84 | ±42.81 | n=197 |
| X 3 | P10 | 1d | 109.22 | ±22.08 | n=267 |
| | | 3d | 185.41 | ±24.61 | n=182 |
| | 722 | 5d | 248.1 | ±37.73 | n=137 |
| X 5 | P11 | 1d | 114.96 | ±26.74 | n=43 |
| | 1099 | 3d | 184.42 | ±26.53 | n=117 |
| | | 5d | 254.52 | ±30.81 | n=111 |
| X 12 | P12 | 1d | 111.99 | ±22.20 | n=114 |
| | 967 | 3d | 194.88 | ±31.00 | n=151 |
| | | 5d | 264.04 | ±44.14 | n=125 |
| X 11 | P13 | 1d | 117.58 | ±25.54 | n=182 |
| | 896 | 3d | 187.45 | ±36.88 | n=181 |
| | | 5d | 269.44 | ±33.77 | n=107 |
| | P14 | 1d | 102.57 | ±22.72 | n=206 |
| | | 5d | 247.11 | ±34.45 | n=145 |
| X 12 | P15 | 1d | 118.58 | ±27.15 | n=186 |
| | 719 | 3d | 195.72 | ±39.69 | n=123 |
| | | 5d | 251.51 | ±44.45 | n=112 |
| X 9 | P16 | 1d | 108.1 | ±22.49 | n=159 |
| | 794 | 3d | 183.99 | ±37.50 | n=116 |
| | | 5d | 221.74 | ±44.37 | n=109 |

**[Table 1-2]**

| **Record of suspension sphere culture of KhES-1 cells in long-term passage (continued)** | | | | | |
|---|---|---|---|---|---|
| split ratio | passage number (left) and sphere number (right) | number of days after passage | average sphere diameter (µm) | standard deviation of sphere diameter (µm) | number of spheres measured |
| X 12 | P17 | 1d | 107.09 | ±21.88 | n=120 |
| | 530 | 3d | 188.35 | ±34.67 | n=137 |
| | | 5d | 242.92 | ±43.92 | n=97 |
| X 7 | P18 | 1d | 111.72 | ±24.97 | n=117 |
| | 909 | 3d | 178.24 | ±36.03 | n=148 |
| | | 5d | 238.09 | ±40.70 | n=138 |
| X 14 | P19 | 1d | 115.97 | ±24.36 | n=142 |
| | 483 | 3d | 196.81 | ±37.70 | n=141 |
| | | 5d | 238.71 | ±38.48 | n=164 |
| X 6 | P20 | 1d | 116.71 | ±21.63 | n=146 |
| | 998 | 3d | 191.23 | ±37.00 | n=189 |
| | | 5d | 232.51 | ±39.31 | n=185 |

By passing the sphere suspension through a 50 µm nylon mesh, the hES cell spheres were mechanically fragmented into highly uniform small spheres (average diameter: about 80 µm) without an enzymatic dissociation. The spheres were cultured for 5 days after passage. As a result, they grew into a sphere size appropriate for the next passage, without undergo ing differentiation or cell death. The number of the hES cells increased to more than 10¹⁶-fold after 20 passages (100 days) from that when the passage culture was started. The average split ratio at passage after the initial two passages was 9.

Although a mesh having a pore size of 70 µm or 100 µm was also tested, the hES cell spheres could not be passage cultured efficiently since the pore was too large.

### Reference

### Example 4: Characterization of hPS cell after long-term passage culture

### (1) hES cell

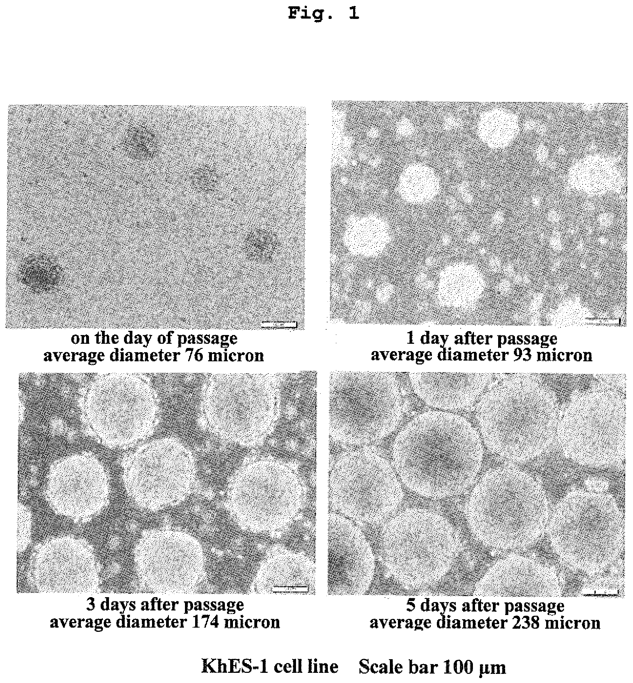

The KhES-1 cell spheres of the 4th passage were observed under a microscope on 0, 1, 3 and 5 days after passage (Fig. 1). The size of the spheres had uniformly increased.

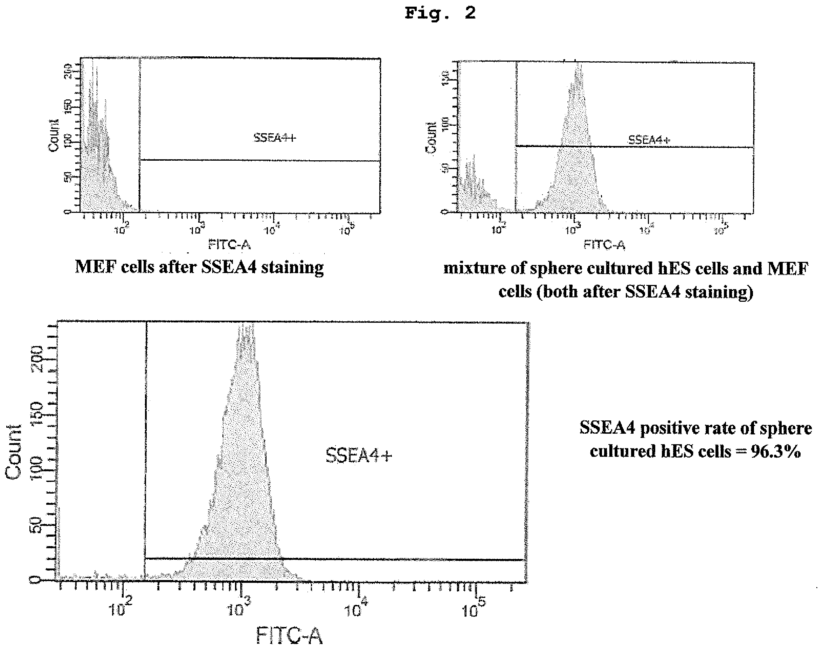

The expression of pluripotency marker (SSEA4) in the hES cells after 11 passages was examined by FACS analysis. 96.3% of the hES cells showed SSEA4 positive (Fig. 2).

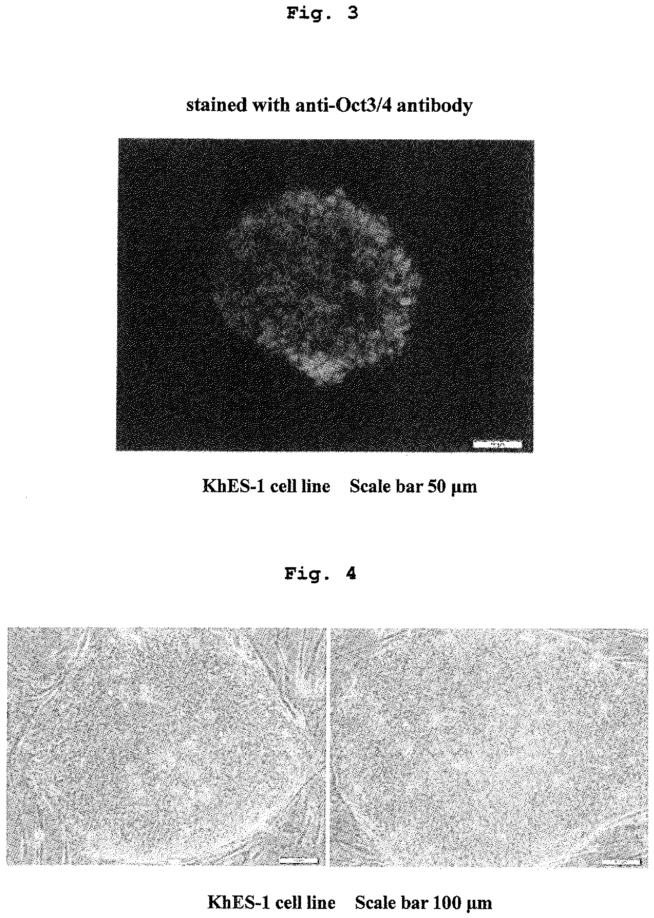

A frozen section of hES cell sphere after 10 passages was prepared, and stained with an anti-Oct3/4 antibody. As a result, the nuclei of all cells were stained (Fig. 3).

These results show that the undifferentiated state of the hES cell is maintained well even after 10 or 11 passages.

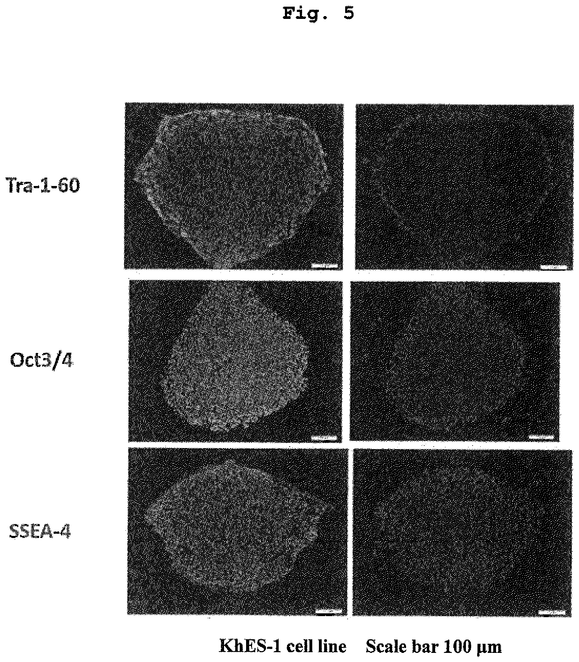

Next, the KhES-1 cells after 18 passages were plated on a feeder cell, and adherent culture was performed. As a result, typical hES cell colonies were formed (Fig. 4). Immunocytostaining of these hES cell colonies for pluripotency markers (Tra-1-60, Oct 3/4, SSEA-4) has clarified that the pluripotency of the hES cells was maintained even after 18 passages (Fig. 5).

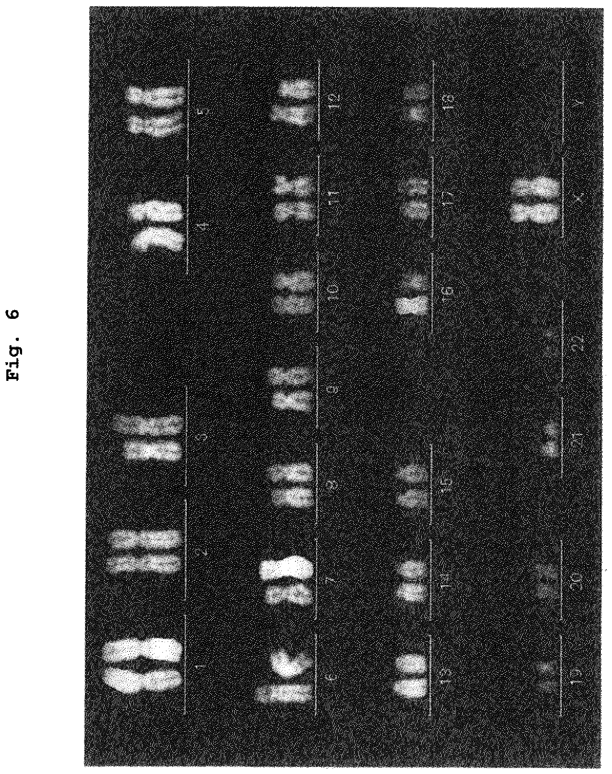

Furthermore, karyotype analysis of KhES-1 cells after 17 passages has revealed that the chromosome of the hES cells was normal even after 17 passages (Fig. 6).

### (2) hiPS cell

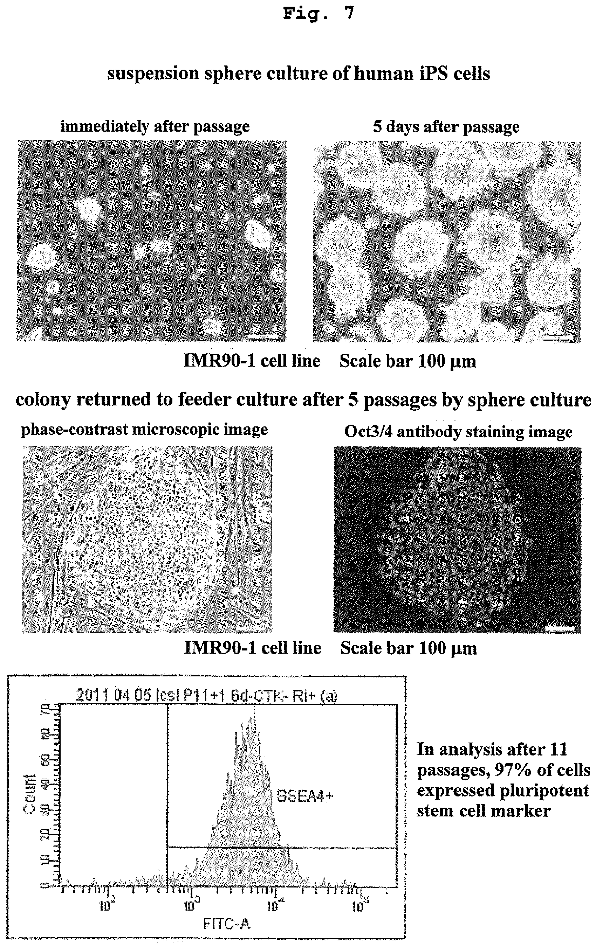

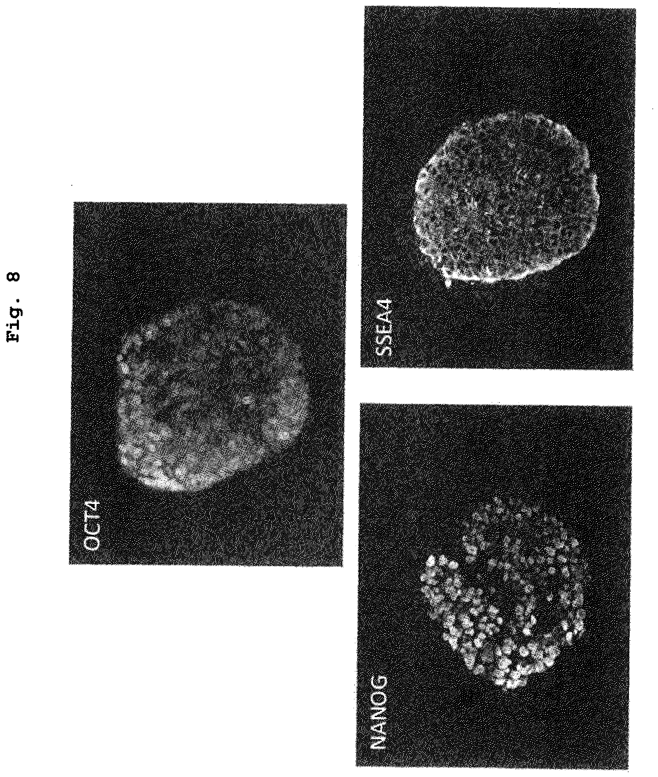

Characterization of hiPS cells (IMR90-1 cell line and 253G1 cell line) cultured according to protocols of suspension culture and passage similar to those used in Example 3 were performed. The results similar to those for hES cells were also obtained for the hiPS cells. That is, IMR90-1 cell spheres after 10 or more passages were observed under a microscope immediately after passage and 5 days after passage. The sphere size had uniformly increased (Fig. 7). In addition, IMR90-1 cells after 5 passages were plated on a feeder cell, and adherent culture was performed. As a result, typical hiPS cell colonies were formed (Fig. 7). Furthermore, the expression of pluripotency marker (SSEA4) in IMR90-1 cells after 11 passages was examined by FACS analysis. 97% of the IMR90-1 cells showed SSEA4 positive (Fig. 7). In addition, a frozen section of the 253G1 cell sphere after 22 passages was prepared, and the expression of undifferentiated state markers (OCT3/4, NANOG, SSEA4) was examined by immunostaining. As a result, undifferentiated property could be confirmed for all cells (Fig. 8).

These results show that the above-mentioned protocols can be utilized not only for the maintenance and amplification of ES cells but also for those of iPS cells.

### Example 5: Analysis for pluripotency of hPS cell after long-term passage culture

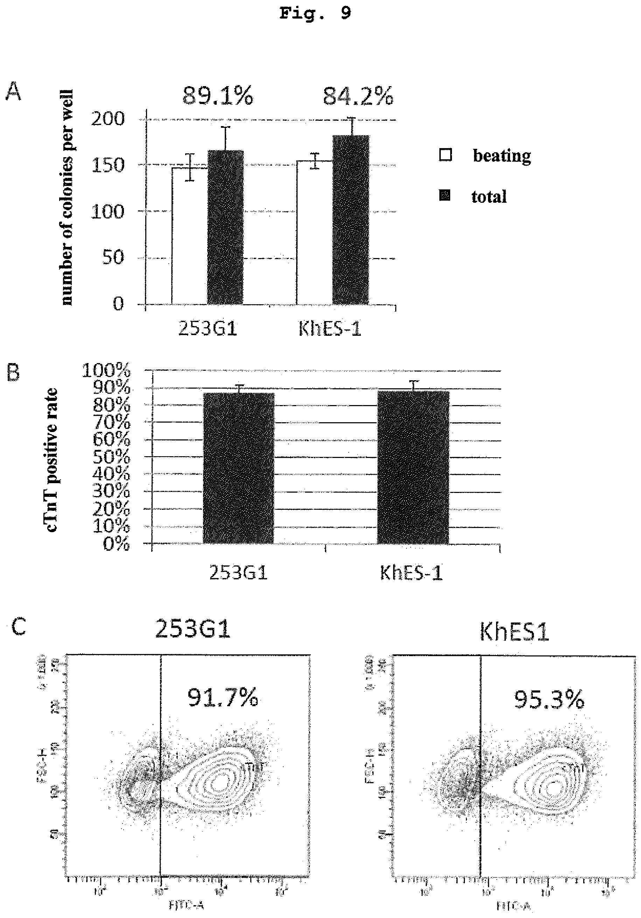

Next, pluripotency of hES cells (reference KhES-1 cell line) and hiPS cells (253G1 cell line) after long-term passage culture was examined. First, the reference KhES-1 cell sphere after 41 passages and 253G1 cell sphere after 10 passages were cultured according to the method described in I. Minami et al. (2012) Cell Reports, in press, to induce differentiation into cardiomyocytes. As a result, 89.1% of 253G1 cell sphere-derived colonies and 84.2% of reference KhES-1 cell sphere-derived colonies were beating (Fig. 9A). As a result of FACS analysis, moreover, about 90% of the cells were positive for myocardial marker cTnT (Figs. 9B, C).

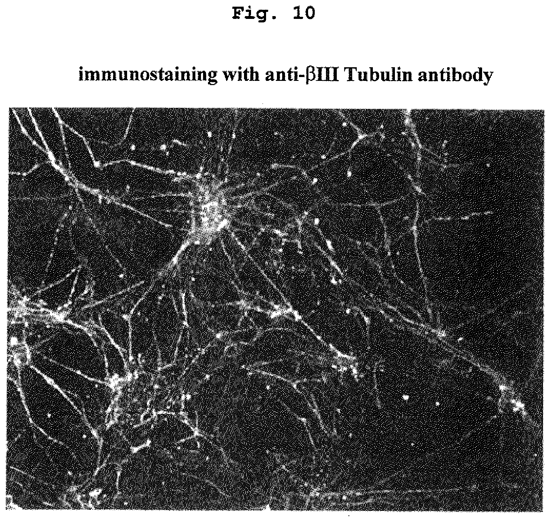

Furthermore, 253G1 cell spheres after 25 passages were cultured according to the method described in K. Sakurai et al. (2010) Nucleic Acids Res., 38: e96 to induce differentiation into neurons. The cells after 32 days from the differentiation induction were double-stained with anti-βIII-Tubulin antibody and DAPI (Fig. 10). As a result, neural cell marker βIII-tubulin positive cells having a neuron-like morphology were confirmed. 89.1% of 253G1 cell sphere-derived colonies and 84.2% of reference KhES-1 cell sphere-derived colonies were beating (Fig. 9A). As a result of FACS analysis, about 90% of the cells were myocardial marker cTnT positive (Figs. 9B, C).

### Example 6: Consideration of conditions of passage and culture after passage using hiPS cells

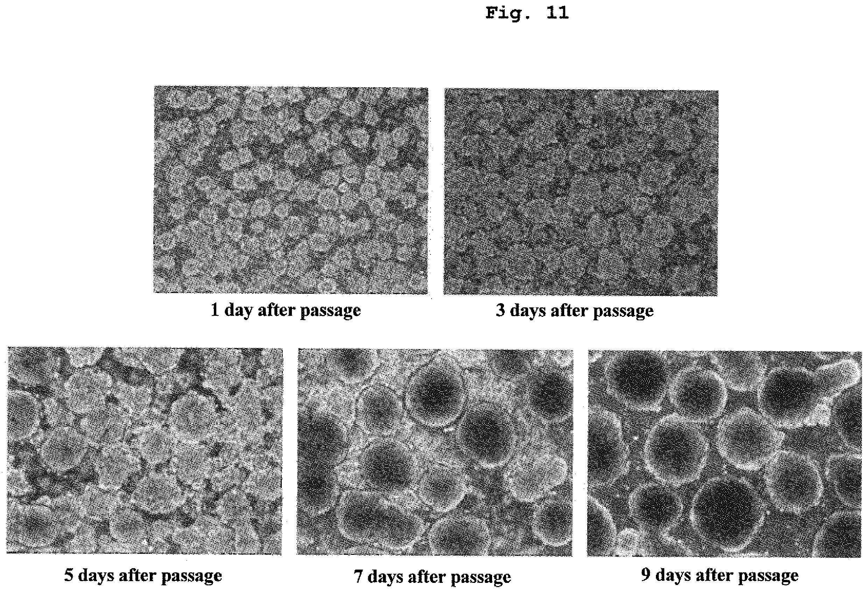

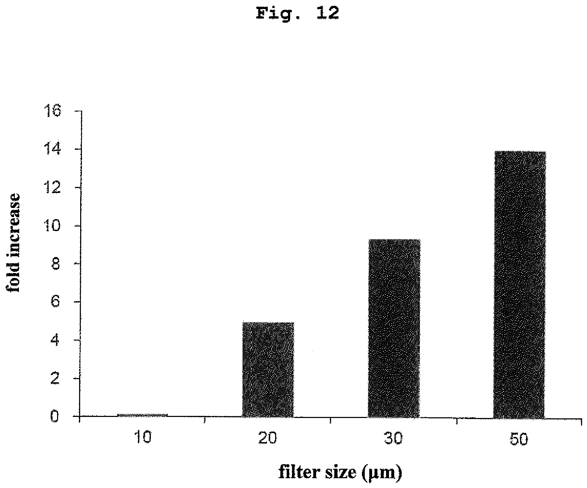

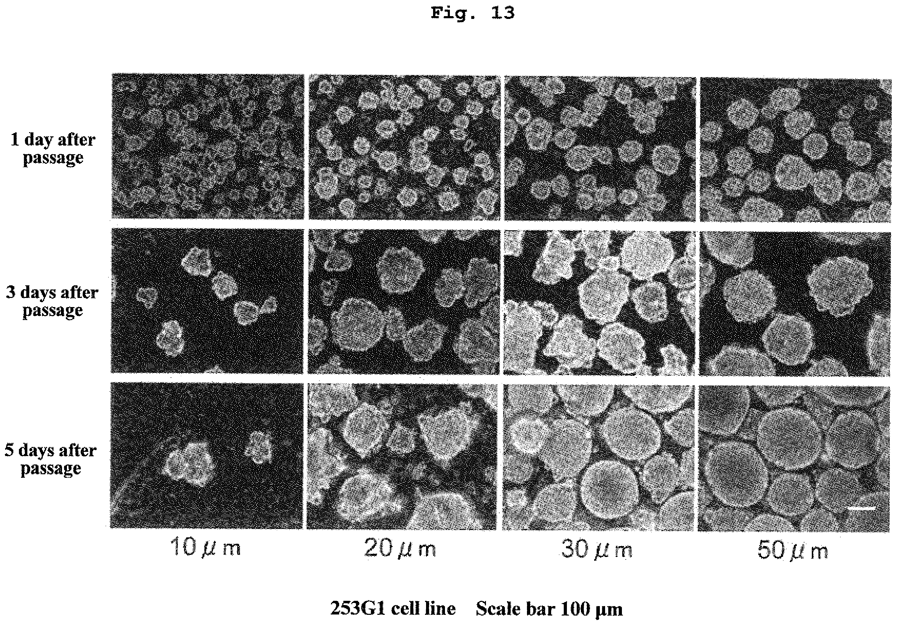

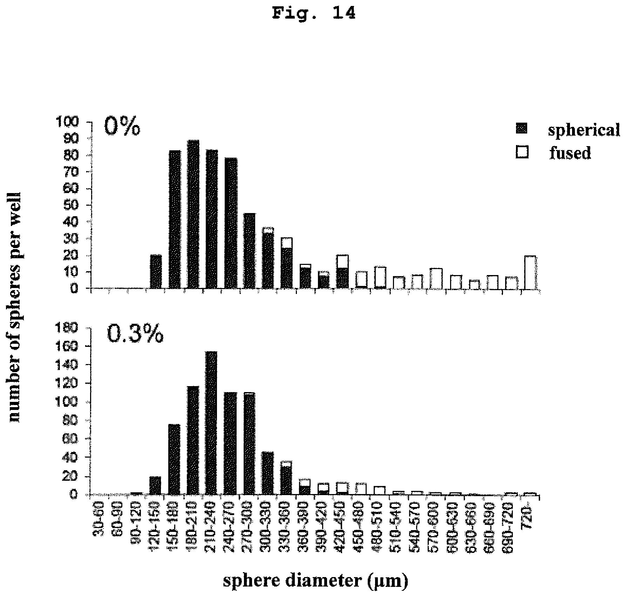

(1) The 253G1 cell spheres of the 24th passage were observed under a microscope 1, 3, 5, 7 and 9 days after passage (Fig. 11). Since a nonuniform structure appeared in the inside on day 7 and thereafter of the passage, the next passage was performed 5 days after passage.
(2) Using various CellTrics filters having different pore sizes (Partec, pore sizes 10, 20, 30 and 50 µm), the effect of mesh size at passage was examined. As a result, it was found that the cell number after 5 days of passage increased most when a mesh having a pore size of 50 µm was used, and the increase rate of the cell number decreased as the mesh size became smaller (Fig. 12). In addition, the morphology of the spheres was observed under a microscope on days 1, 3 and 5 after passage (Fig. 13). As a result, when a mesh having a pore size of 50 µm was used, the cells were amplified in an appropriate size and in a spherical form. However, when a mesh having a pore size of 10 or 20 µm was used, survived spheres were not morphologically spherical.
(3) The effect of methylcellulose for suspension sphere culture of 253G1 cells was examined. 253G1 cells were cultured according to a protocol similar to that used in Example 3 except that medium 1 and medium 2 containing 0.3 w/v% methylcellulose were used. The number of spheres for each sphere size 5 days after 21st passage of 253G1 cell spheres was counted for spherical spheres and fused spheres. Comparison with culture in a methylcellulose-free medium is shown in Fig. 14. Addition of 0.3 w/v% methylcellulose effectively suppressed fusion of spheres.

### Industrial Applicability

An enzyme treatment that loosens adhesion between cells was considered to be indispensable for passage handling of the cells. A mechanical passage method without using an enzyme treatment was considered to cause a large damage on cell aggregates. The present invention is based on a novel idea that destroys existing conventional knowledge. Denial of the need for an enzyme treatment altogether solves the problems of the risk of contamination with virus and the like caused by animal-derived enzymes and high cost from the use of recombinant enzymes. Moreover, the simplicity as evidenced by the passage that can be completed by a single handling, and no requirement for any solution other than a culture medium (e.g., an enzyme solution for dissociation and the like) is extremely useful for incorporation into automated culture systems and the like.

Generally, mesh is used for the removal of cell aggregates remaining after cell dispersion. Therefore, the idea that the mesh itself fragments cell aggregates is completely unpredictable.

Furthermore, a method for increasing the viscosity of a culture medium has conventionally been used to create a microenvironment around non-adherent cells such as hematopoietic cells by preventing diffusion and flow in the culture medium, and it is not often used to prevent adhesion and fusion of cell aggregates. In fact, despite that almost all of about 10 existing papers relating to a suspension culture method point out that adhesion and fusion of cell aggregates is a severe problem and suppression thereof is desired, no paper providing a means to solve the problem has been reported.

The present invention is based on the finding of plural effects unpredictable from the Prior Art, and is extremely simple and effective, as compared to conventional methods, for the design of and incorporation into a mass culture system, particularly an automated culture system, which is indispensable for the practical application of pluripotent stem cells to medicine and drug discovery, and can be utilized for the development of such culture system.

While the present invention has been described with emphasis on preferred embodiments, it is obvious to those skilled in the art that the preferred embodiments can be modified. The present invention intends that the present invention can be embodied by methods other than those described in detail in the present specification. Accordingly, the present invention encompasses all modifications encompassed in the gist and scope of the appended "CLAIMS."

## Claims

1. A method of maintaining and amplifying pluripotent stem cells, comprising repeating the following steps:
(i) suspension culturing pluripotent stem cells until cell aggregates have an average diameter of about 200 - about 300 µm,
(ii) fragmenting the cell aggregates obtained by step (i) into cell aggregates having a uniform average diameter of about 80 - about 120 µm.

2. The method according to claim 1, wherein the suspension culture of the pluripotent stem cells in step (i) is performed until the cell aggregates have an average diameter of about 250 µm, and the fragmentation in step (ii) affords uniform cell aggregates having an average diameter of about 80 µm.

3. The method according to claim 1 or 2, wherein the fragmentation in step (ii) is performed by passing the cell aggregates through a mesh.

4. The method according to claim 3, wherein the pore size of the mesh is about 30 - about 70 µm, preferably about 40 - about 60 µm, more preferably about 50 µm.

5. The method according to any one of claims 1 to 4, wherein the culture in step (i) is performed in a medium containing a water-soluble polymer component having a viscosity that does not cause adhesion of cell aggregates.

6. The method according to claim 5, wherein the water-soluble polymer is selected from polysaccharide or ether thereof, a synthetic hydrogel polymer and a biopolymer, and artificial polymers mimicking them.

7. The method according to claim 5, wherein the water-soluble polymer is methylcellulose or a temperature rise-type thermosensitive hydrogel.

8. The method according to any one of claims 1 to 7, wherein the pluripotent stem cell is an ES cell or an iPS cell.

9. The method according to any one of claims 1 to 8, wherein the pluripotent stem cell is derived from human.

## Patentansprüche

1. Verfahren zur Erhaltung und Vermehrung pluripotenter Stammzellen, umfassend das Wiederholen der folgenden Schritte:
(i) Züchten pluripotenter Stammzellen als Suspension, bis Zellaggregate einen durchschnittlichen Durchmesser von etwa 200 bis etwa 300 µm haben,
(ii) Fragmentieren der aus Schritt (i) erhaltenen Zellaggregate in Zellaggregate, die einen einheitlichen durchschnittlichen Durchmesser von etwa 80 bis etwa 120 µm haben.

2. Verfahren nach Anspruch 1, wobei das Züchten der pluripotenten Stammzellen als Suspension in Schritt (i) durchgeführt wird, bis die Zellaggregate einen durchschnittlichen Durchmesser von etwa 250 µm aufweisen und das Fragmentieren in Schritt (ii) einheitliche Zellaggregate mit einem durchschnittlichen Durchmesser von etwa 80 µm erzeugen.

3. Verfahren nach Anspruch 1 oder 2, wobei das Fragmentieren in Schritt (ii) durchgeführt wird, indem die Zellaggregate durch ein Sieb passiert werden.

4. Verfahren nach Anspruch 3, wobei die Porengröße des Siebs etwa 30 bis etwa 70 µm ist, bevorzugt etwa 40 bis etwa 60 µm ist, stärker bevorzugt etwa 50 µm ist.

5. Verfahren nach einem der Ansprüche 1 bis 4, wobei das Züchten in Schritt (i) in einem Medium durchgeführt wird, das eine wasserlösliche Polymerkomponente enthält, die eine Viskosität hat, die keine Adhäsion von Zellaggregaten hervorruft.

6. Verfahren nach Anspruch 5, wobei das wasserlösliche Polymer ausgewählt ist aus einem Polysaccharid oder Ether davon, einem synthetischen Hydrogelpolymer und einem Biopolymer, und künstlichen Polymeren, die diese nachahmen.

7. Verfahren nach Anspruch 5, wobei das wasserlösliche Polymer Methylcellulose oder ein thermosensitives Temperatur-"rise-type"-Hydrogel (temperature rise-type thermosensitive hydrogel) ist.

8. Verfahren nach einem der Ansprüche 1 bis 7, wobei die pluripotente Stammzelle eine ES-Zelle oder eine iPS-Zelle ist.

9. Verfahren nach einem der Ansprüche 1 bis 8, wobei die pluripotente Stammzelle vom Menschen stammt.

## Revendications

1. Procédé de maintien et d'amplification de cellules souches pluripotentes, comprenant la répétition des étapes suivantes :
(i) la culture en suspension de cellules souches pluripotentes jusqu'à ce que des agrégats cellulaires possèdent un diamètre moyen d'environ 200 - environ 300 µm,
(ii) la fragmentation des agrégats cellulaires obtenus à l'étape (i) en agrégats cellulaires possédant un diamètre moyen uniforme d'environ 80 - environ 120 µm.

2. Procédé selon la revendication 1, dans lequel la culture en suspension des cellules souches pluripotentes à l'étape (i) est réalisée jusqu'à ce que les agrégats cellulaires possèdent un diamètre moyen d'environ 250 µm, et la fragmentation à l'étape (ii) permet d'obtenir des agrégats cellulaires uniformes possédant un diamètre moyen d'environ 80 µm.

3. Procédé selon la revendication 1 ou 2, dans lequel la fragmentation à l'étape (ii) est réalisée par le passage des agrégats cellulaires à travers une maille.

4. Procédé selon la revendication 3, dans lequel la taille de pore de la maille est d'environ 30 - environ 70 µm, de préférence environ 40 - environ 60 µm, de manière davantage préférée environ 50 µm.

5. Procédé selon l'une quelconque des revendications 1 à 4, dans lequel la culture à l'étape (i) est réalisée dans un milieu contenant un composant de polymère hydrosoluble possédant une viscosité qui n'entraîne pas d'adhésion des agrégats cellulaires.

6. Procédé selon la revendication 5, dans lequel le polymère hydrosoluble est sélectionné parmi un polysaccharide ou un éther de celui-ci, un polymère d'hydrogel synthétique et un biopolymère, et des polymères artificiels les imitant.

7. Procédé selon la revendication 5, dans lequel le polymère hydrosoluble est la méthylcellulose ou un hydrogel thermosensible de type « élévation de température ».

8. Procédé selon l'une quelconque des revendications 1 à 7, dans lequel la cellule souche pluripotente est une cellule ES ou une cellule iPS.

9. Procédé selon l'une quelconque des revendications 1 à 8, dans lequel la cellule souche pluripotente est dérivée d'un humain.
